# EUROPEAN PATENT SPECIFICATION

(11) **EP 4 171 475 B1**
(45) Date of publication and mention of the grant of the patent: **19.06.2024**
(21) Application number: 21734865.5
(22) Date of filing: 29.06.2021
(51) Int. Cl.: A61K 8/06, A61K 8/37, A61Q 19/00

(54) **COMPOSITION COMPRISING A COMBINATION OF POLYGLYCEROL ESTERS**
ZUSAMMENSETZUNG MIT EINER KOMBINATION AUS POLYGLYCEROLESTERN
COMPOSITION COMPRENANT UNE COMBINAISON D'ESTERS DE POLYGLYCÉROL

(30) Priority: 30.06.2020 FR 2006913
(43) Date of publication of application: 03.05.2023
(73) Proprietor: L'OREAL, 75008 Paris (FR)
(72) Inventor: FAUGUE, Virginie, 26400 GIGORS ET LOZERON (FR); GUILBAUD, Sophie, 26400 GIGORS ET LOZERON (FR); PIOUD, Noémie, 26400 GIGORS ET LOZERON (FR)
(74) Representative: Cabinet Nony
(86) International application number: PCT/EP2021/067919
(87) International publication number: WO 2022/002978

(56) References cited:
- WO-A2-2013/120823
- WO-A2-2013/120829
- DATABASE GNPD [Online] MINTEL; 21 January 2019 (2019-01-21), anonymous: "Cica Relief Barrier BB Cream SPF 50+ PA++++", XP055783742, Database accession no. 6280381
- DATABASE GNPD [Online] MINTEL; 18 August 2014 (2014-08-18), anonymous: "Multi-Action Beauty Balm SPF 15", XP055783747, Database accession no. 2608581
- DATABASE GNPD [Online] MINTEL; 4 December 2012 (2012-12-04), anonymous: "Anti-Aging BB Cream SPF20", XP055783751, Database accession no. 1935949

## Description

### Technical field

The present invention aims to provide, for the field of caring for and/or making up keratin materials, notably the skin and/or the lips, and in particular the skin, a novel cosmetic composition, in the form of a water-in-oil emulsion, that is very particularly advantageous with regard to its technical performance, notably in terms of viscosity and stability, and the sensations it affords the user during its application to said keratin materials, and in particular to the skin.

For the purposes of the present invention, the term "keratin materials" notably denotes the skin, the lips and/or the eyelashes, in particular the skin and/or the lips, and preferably the skin of the body and/or the face, and more preferentially of the face.

### Prior art

Emulsions may be defined as being heterogeneous systems comprising at least two liquid phases that are immiscible or that have a very low miscibility with one another. In these systems, one of the phases is dispersed in the form of fine droplets in the other phase, so as to observe a mixture that is macroscopically homogenous to the naked eye. Conventionally, the formulator of cosmetic compositions uses emulsified systems that combine an aqueous phase for freshness and a fatty phase for comfort.

The strong point of these systems is enabling the combination, within one and the same composition, of cosmetic ingredients or active agents that have different affinities with respect to these two aqueous and fatty phases, which are immiscible at room temperature. Emulsifying systems of reverse emulsion type (water-in-oil emulsions, the aqueous phase being in a form dispersed in the continuous fatty phase) have many advantages, with regard to the reduced soapy effect but also the good level of coverage and the homogenous appearance that they provide compared to direct emulsions (oil-in-water emulsions).

Their weak point is on the other hand a significant greasy and tacky feeling, and therefore a lack of lightness for the textures obtained.

In order to overcome these drawbacks, it has already been proposed to formulate reverse emulsions comprising a particularly high aqueous phase content, in particular of at least 50.0% by weight, relative to the total weight of the composition. These emulsions are commonly referred to as HIPE (High Internal Phase Emulsion) reverse emulsions.

HIPE reverse emulsions have very particularly advantageous rheological properties, in particular in terms of viscosity, enabling them to be packaged in ajar.

However, the high aqueous phase content of these compositions leads to a high proportion of droplets, which are also larger and therefore closer to one another than in the context of a "conventional" reverse emulsion, comprising an aqueous phase content of strictly less than 50.0% by weight.

There is therefore, for these compositions, an increased risk of coalescence between the droplets, leading to a destabilization of the emulsion.

Combinations of surfactants have already been proposed in the prior art in order to stabilize water-in-oil emulsions of HIPE type, for example in documents WO 2013/120823 and WO 2013/120829.

Nevertheless, these compositions do not prove completely satisfactory, in particular with regard to the sensory effect provided.

Moreover, the preservatives generally used in the cosmetic compositions, in particular those packaged in a jar, also have a destabilizing effect on these HIPE reverse emulsions, owing to their ambiphilic nature, giving them an action at the interface of the droplets of dispersed aqueous phase.

This action at the interface of the droplets additionally gives rise to a reduction in the antimicrobial activity of these preservatives, and therefore an undesirable reduction in the shelf life of the cosmetic compositions thus formulated.

Finally, it is common to use fillers in the cosmetic compositions, such as diffusing fillers or else soft-focus fillers, the purpose of which is to reduce the visibility of the skin relief. However, the introduction of fillers, in particular in a large amount, may also result in a destabilization of said composition and lead to cosmetic properties that are not in accordance with user expectations.

### Disclosure of the invention

Therefore, there remains a need for cosmetic compositions, formulated in the form of water-in-oil emulsions of HIPE type, that combine particularly advantageous technical performance in terms of sensory and rheological properties, while being stable.

There also remains a need to formulate such compositions combining, with the cosmetic active agents, high-performance preservatives, in particular for packaging in a jar, without impairing the advantageous technical performance thereof, in particular in terms of stability. Finally, there also remains a need to have compositions that are compatible with the current consumer demands, notably regarding the environment.

The present invention specifically aims to meet all or some of these needs.

### Summary of the invention

Thus, according to a first of its aspects, the present invention relates to a composition, notably a cosmetic composition, in particular for making up and/or caring for keratin materials, comprising:
- at least one continuous fatty phase;
- at least one aqueous phase at a concentration of at least 50.0% by weight, relative to the total weight of the composition, and dispersed in said fatty phase;
- at least one polyglycerol ester of at least one poly(hydroxystearic acid) and carboxylic acids, derived from the esterification between at least one polyglycerol and (i) at least one poly(hydroxystearic acid); (ii) at least one di- and/or tricarboxylic acid; and (iii) at least one saturated or unsaturated, linear or branched fatty acid having from 6 to 22 carbon atoms;
- at least one ester of polyglycerol comprising from 4 to 15 glycerol units and of at least one linear or branched, saturated or unsaturated fatty acid comprising from 6 to 24 carbon atoms; and
- glyceryl trihydroxystearate,
the polyglycerol ester of at least one poly(hydroxystearic acid) and carboxylic acids/polyglycerol ester of at least one fatty acid weight ratio being strictly greater than 0.7, preferably greater than 1.0.

It is understood that the weight ratio is expressed as active material of the components.

To the knowledge of the inventors, the stabilization of a cosmetic composition in the form of a water-in-oil emulsion of HIPE type by a combination of specific active agents according to the invention, namely at least two polyglycerol esters as defined above, in a specific weight ratio, and of glyceryl trihydroxystearate, has never been described in the prior art. The inventors have surprisingly observed that the combination of at least two polyglycerol esters as defined above, used in a specific weight ratio, and of glyceryl trihydroxystearate, makes it possible to effectively stabilize the water-in-oil emulsion of HIPE type comprising them.

Moreover, the compositions thus obtained have particularly advantageous cosmetic properties, in particular with regard to the sensory effects afforded to the user during the application, notably in terms of reduced greasy and tacky effects, of improved freshness effect or else of absence of soaping effect (white film on application to the skin). Advantageously, a composition according to the invention does not comprise any controversial ingredients, and in particular does not comprise any silicone compounds, such as silicone oils.

A composition according to the invention is used in particular for caring for and/or making up keratin materials, and preferably for caring for keratin materials.

Thus, according to another of its aspects, the invention further relates to a cosmetic process for making up and/or caring for, preferably for caring for, keratin materials, in particular the skin, comprising at least one step of applying a composition according to the invention to said keratin materials.

Other features, variants and advantages of the compositions according to the invention will emerge more clearly on reading the description and the examples that follow.

### Detailed description

### Composition

As stated previously, a composition according to the invention may be cosmetic and/or dermatological, and is preferably cosmetic.

A composition according to the invention is generally suitable for topical application to the skin and thus generally comprises a physiologically acceptable medium, i.e. a medium that is compatible with the skin.

It is preferably a cosmetically acceptable medium, i.e. a medium which has a pleasant color, odor and feel and which does not cause any unacceptable discomfort, i.e. stinging, tautness or redness, liable to discourage the user from applying this composition.

A cosmetic composition according to the invention is in the form of a reverse emulsion of HIPE type, namely comprising a continuous fatty phase and an aqueous phase in a content of at least 50% by weight, dispersed in said fatty phase.

In particular, a composition according to the invention preferably has a viscosity ranging from 0.1 to 10 Pa.s, preferably from 0.5 to 8.0 Pa.s, more preferentially from 2.5 to 7.0 Pa.s. The viscosity of the composition is measured at 25°C using a Rheomat RM100 Touch^{®} (from the company LAMY) equipped with an MS-R4 spindle rotating at a rotational speed of 200 rpm. The measurement is taken after 10 minutes of rotation. The viscosity measurements are taken at most 1 week after production.

A competition according to the invention can be packaged in a jar.

A composition according to the invention may be prepared according to the techniques that are well known to those skilled in the art.

### Polyglycerol esters of a poly(hydroxystearic acid)

As mentioned above, a composition according to the invention comprises at least one polyglycerol ester of at least one poly(hydroxystearic acid) and carboxylic acids, derived from the esterification between at least one polyglycerol and (i) at least one poly(hydroxystearic acid); (ii) at least one di- and/or tricarboxylic acid; and (iii) at least one saturated or unsaturated, linear or branched fatty acid having from 6 to 22 carbon atoms. Mention may more particularly be made of the esters derived from the reaction of a mixture of polyglycerol with (i) at least one poly(hydroxystearic acid) with from 1 to 10, preferably from 2 to 8, more preferentially still from 2 to 5 polyglycerol units (preferably 4 units); (ii) at least one linear or branched aliphatic dicarboxylic acid having from 2 to 16 carbon atoms, preferably from 4 to 14 carbon atoms (preferably sebacic acid); and (iii) at least one saturated or unsaturated, linear or branched fatty acid having from 6 to 22 carbon atoms, preferably from 16 to 20 carbon atoms (preferably isostearic acid). Advantageously, the degree of esterification of the polyglycerol mixture is between 20% and 40%, preferably between 40% and 70%.

According to one preferred embodiment, the polyglycerol ester of a poly(hydroxystearic acid) is derived from the esterification between a mixture of polyglycerol and (i) a poly(hydroxystearic acid) with 4 polyglycerol units; (ii) linear or branched aliphatic dicarboxylic acids having 8 to 12 carbon atoms; and (iii) saturated or unsaturated, linear or branched fatty acids having from 16 to 20 carbon atoms.

As a preferred example of a poly(hydroxystearic acid) ester of polyglycerol, mention may be made of polyglyceryl-4 diisostearate/polyhydroxystearate/sebacate of formula: 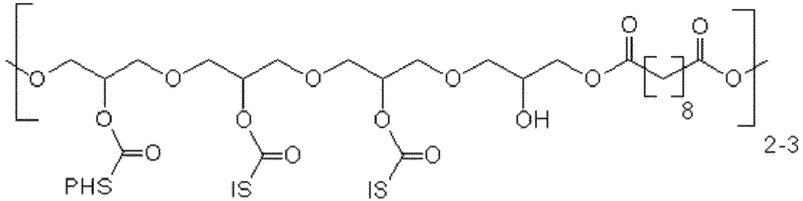 in which PHS denotes polyhydroxystearic acid and IS denotes isostearic acid.

Such a polyglyceryl-4 diisostearate/polyhydroxystearate/sebacate compound is for example sold under the name ISOLAN^{®} GPS by the company EVONIK GOLDSCHMIDT.

The polyglycerol ester of a poly(hydroxystearic acid) and carboxylic acids according to the invention may advantageously be used as a mixture with other esters of polyglycerol, in particular comprising 3 polyglycerol units, and of at least one fatty acid, in particular an oleic acid.

More preferably still, use will be made in the composition of the invention of the Polyglyceryl-4 Diisostearate/Polyhydroxystearate/Sebacate (and) Caprylic/Capric Triglyceride (and) Polyglyceryl-3 Oleate (and) Diisostearoyl Polyglyceryl-3 Dimer Dilinoleate mixture sold under the name ISOLAN^{®} 17 MB by the company EVONIK GOLDSCHMIDT.

A composition according to the invention advantageously comprises between 0.5% and 5.0% by weight, preferably between 1.0% and 4.0% by weight, more preferentially between 1.5% and 3.5% by weight, of at least one polyglycerol ester of a poly(hydroxystearic acid) and carboxylic acids as defined above, relative to the total weight of the composition.

### Polyglycerol fatty acid esters

As mentioned above, a composition according to the invention comprises at least one ester of a polyglycerol comprising from 4 to 15 glycerol units and of at least one, in particular one, linear or branched, saturated or unsaturated fatty acid comprising from 6 to 24 carbon atoms.

Preferably, the polyglycerol fatty acid ester(s) is (are) chosen from esters derived from the reaction of polyglycerol comprising from 4 to 12 glycerol units, preferably from 4 to 10 glycerol units, and of a saturated or unsaturated, linear or branched fatty acid having from 8 to 22 carbon atoms, preferably from 8 to 20 carbon atoms, better still from 12 to 20 carbon atoms, even better still from 16 to 20 carbon atoms.

As examples of fatty acids suitable for the synthesis of the polyglycerol ester of at least one fatty acid according to the invention, mention may be made of isostearic acid, stearic acid, linoleic acid, oleic acid, behenic acid, myristic acid, lauric acid, capric acid and mixtures thereof.

Preferably, the polyglycerol fatty acid ester(s) is (are) chosen from the esters derived from the esterification reaction between a polyglycerol comprising from 4 to 5 glycerol units, in particular comprising 4 glycerol units, and at least one aliphatic monocarboxylic acid comprising an alkyl chain having from 16 to 20 carbon atoms, such as a stearyl and/or isostearyl chain.

Mention may for example be made of the ester derived from the reaction of polyglycerol-4 (glycerol homopolymer comprising 4 glycerol units) and isostearic acid (INCI name: polyglyceryl-4 isostearate) such as the one sold under the name ISOLAN^{®} GI34 by the company EVONIK GOLDSCHMIDT.

A composition according to the invention advantageously comprises between 0.1% and 5.0% by weight, preferably between 0.5% and 4.0% by weight, more preferentially between 1.0% and 4.0% by weight, of at least one polyglycerol fatty acid ester, relative to the total weight of the composition.

According to one embodiment, the polyglycerol esters present in a composition according to the invention, and as defined above, have an HLB, measured at 25°C, of less than or equal to 8.0, preferably between 0.1 and 8.0, more preferentially between 2.0 and 8.0.

The HLB (hydrophilic-lipophilic balance) is the ratio between the hydrophilic part and the lipophilic part in the surfactant molecule. This time is well known to those skilled in the art and is described for example in "The HLB system, A time-saving guide to Emulsifier Selection", published by ICI Americas Inc, 1984. The HLB of the surfactant(s) used according to the invention may be determined via the Griffin method or the Davies method.

As mentioned above, the polyglycerol ester of at least one poly(hydroxystearic acid) and carboxylic acids/polyglycerol ester of at least one fatty acid weight ratio is strictly greater than 0.7. It is preferably greater than or equal to 0.8, more preferentially greater than or equal to 1. In particular, the polyglycerol ester of at least one poly(hydroxystearic acid) and carboxylic acids/polyglycerol ester of at least one fatty acid weight ratio may be between 0.8 and 2.0.

### Glyceryl trihydroxystearate

As mentioned above, a composition according to the invention comprises glyceryl trihydroxystearate (INCI name: trihydroxy stearin).

### Glyceryl trihydroxystearate is a compound of formula (I):

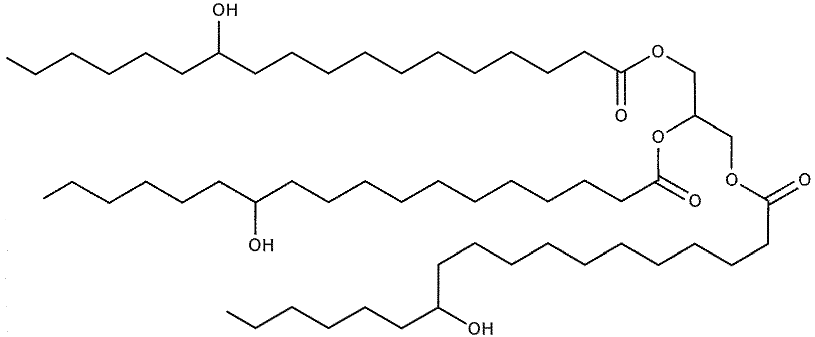

It is a wax, namely a lipophilic compound, which is solid at room temperature (25°C), with a reversible solid/liquid change of state, which has a melting point of greater than or equal to 30°C, in particular greater than 60°C.

For the purposes of the invention, the melting point corresponds to the temperature of the most endothermic peak observed on thermal analysis (DSC) as described in the standard ISO 11357-3; 1999. The melting point of the wax may be measured using a differential scanning calorimeter (DSC), for example the calorimeter sold under the name MDSC 2920 by the company TA Instruments.

A composition according to the invention advantageously comprises between 0.01% and 2.0% by weight, in particular between 0.1% and 1.5% by weight, preferably between 0.1% and 1% by weight, more preferentially between 0.2% and 0.8% by weight, of glyceryl trihydroxystearate, relative to the total weight of the composition.

### Fatty phase

As mentioned above, a composition according to the invention comprises at least one continuous fatty phase.

The fatty phase preferably contains at least one oil, notably a cosmetic oil. It may also contain other fatty substances.

The term "oil" means a water-immiscible non-aqueous compound that is liquid at room temperature (20°C) and at atmospheric pressure (760 mmHg).

A fatty phase that is suitable for preparing the compositions, notably cosmetic compositions, according to the invention may comprise hydrocarbon-based oils, silicone oils, fluoro oils or non-fluoro oils, or mixtures thereof.

Preferably, a composition according to the invention comprises less than 2.0% by weight of silicone oil(s), in particular less than 1.0% by weight of silicone oil(s), preferably less than 0.5% by weight of silicone oil(s), relative to the total weight of the composition, and more preferentially is free of silicone oil(s).

Thus, according to a particular embodiment, a composition according to the invention is free of silicone oil(s), in particular free of cyclopentasiloxane.

A composition comprising a limited content of silicone oil(s) is advantageously more natural, but also lighter, less tacky and less rough to the touch, with a softer finish, than a composition comprising 2% by weight or more of silicone oil(s), relative to the total weight of the composition.

The oils may be volatile or nonvolatile.

They may be of animal, plant, mineral or synthetic origin.

The term "nonvolatile" refers to an oil of which the vapor pressure at room temperature and atmospheric pressure is non-zero and is less than 10⁻³ mmHg (0.13 Pa).

For the purposes of the present invention, the term "silicone oil" means an oil comprising at least one silicon atom, and notably at least one Si-O group.

The term "fluoro oil" means an oil comprising at least one fluorine atom.

The term "hydrocarbon-based oil" means an oil mainly containing hydrogen and carbon atoms.

The oils may optionally comprise oxygen, nitrogen, sulfur and/or phosphorus atoms, for example in the form of hydroxyl or acid radicals.

For the purposes of the invention, the term "volatile oil" means any oil that is capable of evaporating on contact with the skin in less than one hour, at room temperature and atmospheric pressure. The volatile oil is a volatile cosmetic compound, which is liquid at room temperature, in particular having a non-zero vapor pressure, at room temperature and atmospheric pressure, in particular having a vapor pressure ranging from 0.13 Pa to 40 000 Pa (10⁻³ to 300 mmHg), in particular ranging from 1.3 Pa to 13 000 Pa (0.01 to 100 mmHg), and more particularly ranging from 1.3 Pa to 1300 Pa (0.01 to 10 mmHg).

Mention may in particular be made of volatile hydrocarbon-based oils containing from 8 to 16 carbon atoms, branched C₈-C₁₆ alkanes, for instance C₈-C₁₆ isoalkanes (also known as isoparaffins), isododecane, isodecane, isohexadecane and, for example, the oils sold under the trade names Isopar or Permethyl, branched C₈-C₁₆ esters, for instance isohexyl neopentanoate, and mixtures thereof. In particular, the volatile hydrocarbon-based oil is chosen from volatile hydrocarbon-based oils containing from 8 to 16 carbon atoms and mixtures thereof.

Mention may also be made of volatile linear alkanes comprising from 8 to 16 carbon atoms, in particular from 10 to 15 carbon atoms and more particularly from 11 to 13 carbon atoms, for instance n-dodecane (C₁₂) and n-tetradecane (C₁₄) sold by Sasol under the respective references Parafol^{®} 12-97 and Parafol^{®} 14-97, and also mixtures thereof, the undecane-tridecane mixture, mixtures of n-undecane (C₁₁) and of n-tridecane (C₁₃) obtained in examples 1 and 2 of patent application WO 2008/155 059 from the company Cognis, and mixtures thereof.

Mention may also be made of the following mixtures of linear or branched alkanes, preferably of plant origin:
- a mixture of C₁₅-C₁₉ branched alkanes, for example that which is sold by the company SEPPIC under the name Emogreen^{®} L15;
- a mixture of C₁₅-C₁₉ linear and/or branched alkanes, for example that which is sold by the company SEPPIC under the name Emogreen^{®} L19.

Preferably, a composition according to the invention may comprise at least one hydrocarbon-based oil chosen from volatile linear alkanes comprising from 11 to 13 carbon atoms, in particular an undecane-tridecane mixture, and linear and/or branched C₁₅-C₁₉ alkanes, in particular a mixture of linear and/or branched C₁₅-C₁₉ alkanes.

In particular, such hydrocarbon-based oils may be present in a composition according to the invention in a content ranging from 3.0% to 25.0% by weight and preferably from 7.0% to 20.0% by weight, relative to the total weight of the composition.

Volatile silicone oils that may be mentioned include volatile linear silicone oils such as hexamethyldisiloxane, octamethyltrisiloxane, decamethyltetrasiloxane, tetradecamethylhexasiloxane, hexadecamethylheptasiloxane and dodecamethylpentasiloxane.

Volatile cyclic silicone oils that may be mentioned include hexamethylcyclotrisiloxane, octamethylcyclotetrasiloxane, decamethylcyclopentasiloxane, cyclohexasiloxane and dodecamethylcyclohexasiloxane, and in particular cyclohexasiloxane.

Mention may also be made of nonvolatile hydrocarbon-based, fluoro and/or silicone oils. Nonvolatile hydrocarbon-based oils that may in particular be mentioned include:
- hydrocarbon-based oils of animal origin,
- hydrocarbon-based oils of plant origin, such as squalane, synthetic ethers having from 10 to 40 carbon atoms, such as dicapryl ether,
- synthetic esters, for instance the oils of formula R₁COOR₂, wherein R₁ represents a linear or branched fatty acid residue including from 1 to 40 carbon atoms and R₂ represents a hydrocarbon-based chain that is in particular branched, containing from 1 to 40 carbon atoms, on condition that R₁ + R₂ is greater than or equal to 10. The esters may notably be chosen from esters of alcohol and of fatty acid, for instance cetostearyl octanoate, esters of isopropyl alcohol, such as isopropyl myristate, isopropyl palmitate, ethyl palmitate, 2-ethylhexyl palmitate, isopropyl stearate, octyl stearate, hydroxylated esters, for instance isostearyl lactate, octyl hydroxystearate, alcohol or polyalcohol ricinoleates, hexyl laurate, neopentanoic acid esters, for instance isodecyl neopentanoate, isotridecyl neopentanoate, isononanoic acid esters, for instance isononyl isononanoate, isotridecyl isononanoate and octyl isononanoate, oleyl erucate, isopropyl lauroyl sarcosinate, diisopropyl sebacate, isocetyl stearate, isodecyl neopentanoate or isostearyl behenate;

- polyol esters and pentaerythritol esters, for instance dipentaerythrityl tetrahydroxystearate/tetraisostearate,
- fatty alcohols that are liquid at room temperature, with a branched and/or unsaturated carbon-based chain containing from 12 to 26 carbon atoms, for instance 2-octyldodecanol, isostearyl alcohol and oleyl alcohol,
- C₁₂-C₂₂ higher fatty acids, such as oleic acid, linoleic acid, linolenic acid, and mixtures thereof,
- carbonates, such as dicaprylyl carbonate,
- nonphenylated silicone oils, such as, for example, caprylyl methicone, and
- phenyl silicone oils, for instance phenyl trimethicones, phenyl dimethicones, phenyltrimethylsiloxydiphenylsiloxanes, diphenyl dimethicones, diphenylmethyldiphenyltrisiloxanes and 2-phenylethyl trimethylsiloxysilicates, dimethicones or phenyl trimethicone with a viscosity of less than or equal to 100 cSt, trimethylpentaphenyltrisiloxane, and mixtures thereof;
and also mixtures of these various oils.

In particular, the composition may also comprise at least one nonvolatile oil, chosen in particular from nonvolatile apolar hydrocarbon-based oils.

For the purposes of the present invention, the term "apolar oil" means an oil whose solubility parameter at 25°C, δₐ, is equal to 0 (J/cm³)^{½}.

The definition and calculation of the solubility parameters in the Hansen three-dimensional solubility space are described in the article by C. M. Hansen: "The three dimensional solubility parameters", J. Paint Technol. 39, 105 (1967).

According to this Hansen space:
- δ_{D} characterizes the London dispersion forces derived from the formation of dipoles induced during molecular impacts;
- δₚ characterizes the Debye interaction forces between permanent dipoles and also the Keesom interaction forces between induced dipoles and permanent dipoles;
- δₕ characterizes the specific interaction forces (such as hydrogen bonding, acid/base, donor/acceptor, etc.); and
- δₐ is determined by the equation δₐ = (δp² + δh²)^{½}.

The parameters δₚ, δₕ, δ_{D} and δₐ are expressed in (J/cm³)^{½}.

The nonvolatile apolar hydrocarbon-based oil is free of oxygen atoms.

Preferably, the nonvolatile apolar hydrocarbon-based oil may be chosen from linear or branched hydrocarbons of mineral or synthetic origin. In particular, it may be chosen from:
- liquid paraffin or derivatives thereof,
- liquid petroleum jelly,
- polybutylenes, for example Indopol H-100 (molar mass or Mw = 965 g/mol), Indopol H-300 (Mw = 1340 g/mol) and Indopol H-1500 (Mw = 2160 g/mol) sold or manufactured by the company Amoco,
- polyisobutenes and hydrogenated polyisobutenes, for example Parleam^{®} sold by the company Nippon Oil Fats, Panalane H-300 E sold or manufactured by the company Amoco (Mw = 1340 g/mol), Viseal 20000 sold or manufactured by the company Synteal (Mw = 6000 g/mol) and Rewopal PIB 1000 sold or manufactured by the company Witco (Mw = 1000 g/mol),
- decene/butene copolymers, polybutene/polyisobutene copolymers, for example Indopol L-14,
- polydecenes and hydrogenated polydecenes, for example Puresyn 10 (Mw = 723 g/mol) and Puresyn 150 (Mw = 9200 g/mol) sold or manufactured by the company Mobil Chemicals,
- and mixtures thereof.

Said nonvolatile oil may also be an ester oil, in particular containing between 18 and 70 carbon atoms.

Examples that may be mentioned include monoesters, diesters or triesters.

The ester oils may in particular be hydroxylated.

The nonvolatile ester oil may preferably be chosen from:
- monoesters comprising between 18 and 40 carbon atoms in total, in particular the monoesters of formula R₁COOR₂ wherein R₁ represents a linear or branched fatty acid residue including from 4 to 40 carbon atoms and R₂ represents a hydrocarbon-based chain that is in particular branched, containing from 4 to 40 carbon atoms, on condition that R₁ + R₂ is greater than or equal to 18, for instance Purcellin oil (cetostearyl octanoate), isononyl isononanoate, C₁₂ to C₁₅ alcohol benzoate, 2-ethylhexyl palmitate, octyldodecyl neopentanoate, 2-octyldodecyl stearate, 2-octyldodecyl erucate, isostearyl isostearate, diisopropyl sebacate, 2-octyldodecyl benzoate, alcohol or polyalcohol octanoates, decanoates or ricinoleates, isopropyl myristate, isopropyl palmitate, butyl stearate, hexyl laurate, 2-ethylhexyl palmitate, 2-hexyldecyl laurate, 2-octyldecyl palmitate, 2-octyldodecyl myristate, 2-diethylhexyl succinate or isoamyl laurate. Preferably, they are esters of formula R₁COOR₂ wherein R₁ represents a linear or branched fatty acid residue including from 4 to 40 carbon atoms and R₂ represents a hydrocarbon-based chain that is in particular branched, containing from 4 to 40 carbon atoms, R₁ and R₂ being such that R₁ + R₂ is greater than or equal to 18. Preferably, the ester comprises between 18 and 40 carbon atoms in total. Preferred monoesters that may be mentioned include isononyl isononanoate, oleyl erucate and/or 2-octyldodecyl neopentanoate;

- diesters, in particular comprising between 18 and 60 carbon atoms in total, in particular between 18 and 50 carbon atoms in total. It is in particular possible to use diesters of dicarboxylic acids and of monoalcohols, preferably such as diisostearyl malate, or glycol diesters of monocarboxylic acids, such as neopentyl glycol diheptanoate or polyglyceryl-2 diisostearate, in particular such as the compound sold under the trade reference Dermol DGDIS by the company Akzo;
- triesters, in particular comprising between 35 and 70 carbon atoms in total, in particular such as triesters of tricarboxylic acids, such as triisostearyl citrate, or tridecyl trimellitate, or glycol triesters of monocarboxylic acids such as polyglyceryl-2 triisostearate;
- tetraesters, in particular with a total carbon number ranging from 35 to 70, such as pentaerythritol or polyglycerol tetraesters of a monocarboxylic acid, for instance pentaerythrityl tetrapelargonate, pentaerythrityl tetraisostearate, pentaerythrityl tetraisononanoate, glyceryl tris(2-decyl)tetradecanoate, polyglyceryl-2 tetraisostearate or else pentaerythrityl tetrakis(2-decyl)tetradecanoate;
- polyesters obtained by condensation of unsaturated fatty acid dimer and/or trimer and of diol, such as those described in patent application FR 0 853 634, in particular such as of dilinoleic acid and of 1,4-butanediol. Mention may in particular be made in this respect of the polymer sold by Biosynthis under the name Viscoplast 14436H (INCI name: dilinoleic acid/butanediol copolymer), or else copolymers of polyols and of diacid dimers, and esters thereof, such as Hailuscent ISDA;
- esters and polyesters of diol dimer and of monocarboxylic or dicarboxylic acid, such as esters of diol dimer and of fatty acid and esters of diol dimer and of dicarboxylic acid dimer, in particular which may be obtained from a dicarboxylic acid dimer derived in particular from the dimerization of an unsaturated fatty acid notably an unsaturated C₈ to C₃₄, notably C₁₂ to C₂₂, in particular C₁₆ to C₂₀ and more particularly C₁₈ fatty acid, such as esters of dilinoleic diacids and of dilinoleic diol dimers, for instance those sold by the company Nippon Fine Chemical under the trade names Lusplan DD-DA5^{®} and DD-DA7^{®};
- vinylpyrrolidone/1-hexadecene copolymers, for instance the product sold under the name Antaron V-216 (also known as Ganex V216) by the company ISP (Mw = 7300 g/mol);
- hydrocarbon-based plant oils such as fatty acid triglycerides (which are liquid at room temperature), in particular of fatty acids containing from 7 to 40 carbon atoms, such as heptanoic or octanoic acid triglycerides or jojoba oil; mention may be made in particular of saturated triglycerides such as caprylic/capric triglyceride, glyceryl triheptanoate, glyceryl trioctanoate, and C₁₈₋₃₆ acid triglycerides such as those sold under the reference DUB TGI 24 by Stéarinerie Dubois; and unsaturated triglycerides such as castor oil, olive oil, ximenia oil and pracaxi oil;
- and mixtures thereof.

Preferably, a composition according to the invention may comprise at least one nonvolatile hydrocarbon-based oil chosen from synthetic ethers having from 10 to 40 carbon atoms, such as dicaprylyl ether, carbonates, such as dicaprylyl carbonate, fatty acid triglycerides, in particular that are saturated, such as caprylic/capric triglyceride, fatty alcohols that are liquid at room temperature, with a branched and/or unsaturated carbon-based chain containing from 12 to 26 carbon atoms, for instance 2-octyldodecanol, and the esters of formula R₁COOR₂ wherein R₁ represents a linear or branched fatty acid residue including from 4 to 40 carbon atoms and R₂ represents a hydrocarbon-based chain that is in particular branched, containing from 4 to 40 carbon atoms, R₁ and R₂ being such that R₁ + R₂ is greater than or equal to 18, such as isoamyl laurate.

More preferentially, a composition according to the invention may comprise at least one nonvolatile hydrocarbon-based oil chosen from synthetic ethers having from 10 to 40 carbon atoms, such as dicaprylyl ether, carbonates, such as dicaprylyl carbonate, fatty acid triglycerides, in particular that are saturated, such as caprylic/capric triglyceride, and mixtures thereof.

In particular, such nonvolatile hydrocarbon-based oils may be present in a composition according to the invention in a content ranging from 6.0% to 25.0% by weight, preferably from 12.0% to 20.0% by weight, relative to the total weight of the composition.

The other fatty substances that may be present in the oily phase are, for example, fatty acids including from 8 to 30 carbon atoms, for instance stearic acid, lauric acid, palmitic acid and oleic acid; waxes, different from glyceryl trihydroxystearate, for instance lanolin, beeswax, carnauba wax or candelilla wax, paraffin wax, lignite wax or microcrystalline waxes, ceresin or ozokerite, and synthetic waxes, for instance polyethylene waxes and Fischer-Tropsch waxes; silicone resins such as trifluoromethyl-C₁-C₄-alkyl dimethicone and trifluoropropyl dimethicone; and silicone elastomers, for instance the products sold under the name KSG by the company Shin-Etsu, under the name Trefil or BY29 by the company Dow Corning, or under the name Gransil by the company Grant Industries.

As fatty substances, a composition according to the invention may preferably comprise at least one fatty alcohol wax.

Such waxes may be chosen from lauryl alcohol, myristyl alcohol, cetyl alcohol, stearyl alcohol, arachidyl alcohol, behenyl alcohol, lignoceryl alcohol, ceryl alcohol, montanyl alcohol, myricyl alcohol, and mixtures thereof.

Preferentially, the fatty alcohol wax is cetyl alcohol.

As fatty substance, a composition according to the invention may preferably comprise at least one butter, in particular a plant butter.

The plant butter(s) suitable for use in the invention is (are) preferably chosen from the group comprising avocado butter, cocoa butter, shea butter, kokum butter, mango butter, murumuru butter, coconut butter, apricot kernel butter, sal butter and urucum butter, and mixtures thereof, and in particular is shea butter.

These fatty substances may be chosen in a varied manner by a person skilled in the art in order to prepare a composition having the desired properties, for example in terms of consistency or texture.

Preferably, a composition according to the invention comprises a fatty phase containing at least one fatty substance.

According to a preferred embodiment, a composition according to the invention comprises at least one nonvolatile hydrocarbon-based oil, and preferably at least one apolar hydrocarbon-based oil.

Preferably, a composition according to the invention comprises at least one, in particular two, nonvolatile hydrocarbon-based oils and one nonvolatile ester oil.

Preferably, a composition according to the invention comprises at least one oil chosen from hydrocarbon-based oils of plant origin, and in particular at least one mixture of linear or branched alkanes.

According to a particularly preferred embodiment, a composition according to the invention also comprises at least one solid fatty substance, in particular a fatty alcohol wax and/or a butter.

Preferably, the fatty phase content is between 5.0% and 50% by weight, in particular between 10% and 30% by weight, and preferably between 15% and 25% by weight, relative to the total weight of the composition.

### Aqueous phase

As mentioned above, a composition according to the invention comprises at least one aqueous phase at a concentration of at least 50.0% by weight, relative to the total weight of the composition, and dispersed in the fatty phase defined above.

The aqueous phase comprises water and optionally a water-soluble solvent.

According to the present invention, the term "water-soluble solvent" denotes a compound that is liquid at room temperature and water-miscible (miscibility with water of greater than 50% by weight at 25°C and atmospheric pressure).

A water that is suitable for use in the invention may be a floral water such as cornflower water and/or a mineral water such as Vittel water, Lucas water or La Roche Posay water and/or a thermal spring water.

The water-soluble solvents which can be used in the composition of the invention can in addition be volatile.

Among the water-soluble solvents that may be used in the composition according to the invention, mention may be made in particular of lower monoalcohols containing from 1 to 5 carbon atoms such as ethanol and isopropanol, glycols containing from 2 to 8 carbon atoms such as ethylene glycol, hexylene glycol, propylene glycol, 1,3-butylene glycol and dipropylene glycol, C₃ and C₄ ketones and C₂-C₄ aldehydes.

According to an alternative embodiment, the aqueous phase of a composition according to the invention may comprise at least one C₂-C₃₂ polyol.

Within the meaning of the present invention, the term "polyol" should be understood as meaning any organic molecule comprising at least two free hydroxyl groups.

Such polyols are more particularly known for giving the composition increased properties in terms of moisturization. Specifically, these compounds are capable of penetrating into the stratum corneum and of keeping it moisturized.

Preferably, a polyol in accordance with the present invention is present in liquid form at room temperature.

A polyol that is suitable for use in the invention may be a compound of linear, branched or cyclic, saturated or unsaturated alkyl type, bearing on the alkyl chain at least two -OH functions, in particular at least three -OH functions and more particularly at least four -OH functions.

The polyols that are suitable for formulating a composition according to the present invention are in particular those notably containing from 2 to 32 carbon atoms, preferably from 3 to 16 carbon atoms.

The polyol may be chosen, for example, from ethylene glycol, pentaerythritol, trimethylolpropane, propylene glycol, 1,3-propanediol, butylene glycol, isoprene glycol, pentylene glycol, hexylene glycol, caprylyl glycol, glycerol, polyglycerols, such as glycerol oligomers, for instance diglycerol, polyethylene glycols, and mixtures thereof.

According to a preferred embodiment of the invention, said polyol is chosen from ethylene glycol, pentaerythritol, trimethylolpropane, propylene glycol, dipropylene glycol, caprylyl glycol, glycerol, polyglycerols, polyethylene glycols, and mixtures thereof.

According to a particular embodiment of the invention, the composition of the invention may comprise at least one polyol, in particular chosen from glycerin, propanediol, and mixtures thereof.

When they are present, the polyol(s) is (are) preferably present in a composition according to the invention in a content ranging from 1.0% to 20% by weight, better still from 3.0% to 15% by weight, preferably from 5.0% to 10% by weight, relative to the total weight of said composition.

The aqueous phase may also comprise stabilizers, for example sodium chloride, magnesium dichloride or magnesium sulfate.

The aqueous phase may also comprise any water-soluble or water-dispersible compound that is compatible with an aqueous phase, such as gelling agents, film-forming polymers, thickeners or surfactants, and mixtures thereof.

Preferably, the aqueous phase is present in a composition according to the invention in a content ranging from 50% to 90% by weight, preferably from 60% to 85% by weight, and more preferentially from 65% to 75% by weight, relative to the total weight of said composition.

### Additives

### Preservatives

A composition according to the invention may also comprise at least one preservative.

Of course, the preservative(s) optionally present in a composition according to the invention is (are) chosen so as not to impair the properties of the composition, in particular in terms of stability.

Preservatives may be used in order to reduce microbial proliferation and to increase the shelf life of the compositions, notably cosmetic compositions, in particular that are packaged in a jar.

The preservatives may be chosen from antibiotics, antifungal agents and mixtures thereof.

Such preservatives are for example alcohols, glycols, parabens, benzoic acids, sodium benzoate, sorbic acid, potassium sorbate, salicylic acid, sodium salicylate, phenoxyethanol or else chlorphenesin.

More generally, these preservatives are described in the publication "International Cosmetic Ingredient Dictionary and Handbook", Seventh Edition, volume 2, pages 1654 and 1655.

Antimicrobial agents that may be mentioned include, for example, the following active agents: β-lactam derivatives, quinolone derivatives, ciprofloxacin, norfloxacin, tetracycline and its salts (hydrochloride), erythromycin and its salts (of zinc, estolate, stearate), amikacin and its salts (sulfate), 2,4,4'-trichloro-2'-hydroxydiphenyl ether (triclosan), 3,4,4'-trichlorobanilide (tricarban), phenoxyethanol, phenoxypropanol, phenoxyisopropanol, doxycycline and its salts (hydrochloride), capreomycin and its salts (sulfate), chlorhexidine and its salts (gluconate, hydrochloride), chlortetracycline and its salts (hydrochloride), oxytetracycline and its salts (hydrochloride), clindamycin and its salts (hydrochloride), ethambutol and its salts (hydrochloride), hexamidine and its salts (isethionate), metronidazole and its salts (hydrochloride), pentamidine and its salts (hydrochloride), gentamicin and its salts (sulfate), kanamycin and its salts (sulfate), lincomycin and its salts (hydrochloride), methacycline and its salts (hydrochloride), methenamine and its salts (hippurate, mandelate), minocycline and its salts (hydrochloride), neomycin and its salts (sulfate), netilmicin and its salts (sulfate), paromomycin and its salts (sulfate), streptomycin and its salts (sulfate), tobramycin and its salts (sulfate), miconazole and its salts (hydrochloride), amantadine and its salts (sulfate, hydrochloride), octopirox, parachlorometaxylenol, nystatin, tolnaftate, zinc pyrithione, clotrimazole, salicylic acid, 5-n-octanoyl salicylic acid (or capryloyl salicylic acid), benzoyl peroxide, 3-hydroxybenzoic acid, glycolic acid, lactic acid, 4-hydroxybenzoic acid, acetylsalicylic acid, 2-hydroxybutanoic acid, 2-hydroxypentanoic acid, 2-hydroxyhexanoic acid, phytic acid, N-acetyl-L-cysteine, lipoic acid, azelaic acid, arachidonic acid, ibuprofen, naproxen, hydrocortisone, acetaminophen, resorcinol, lidocaine hydrochloride, neomycin sulfate, octoxyglycerin, octanoylglycine (or capryloyl glycine), caprylyl glycol (1,2-octanediol), 10-hydroxy-2-decenoic acid, and mixtures thereof.

According to a preferred embodiment, a composition according to the invention comprises a mixture of preservatives comprising at least one alcohol, preferably ethanol, at least one glycol, preferably pentylene glycol, salicylic acid, potassium sorbate and phytic acid.

A composition according to the invention may comprise between 1.0% and 12% by weight of preservatives, preferably between 2.0% and 9.0%, more preferentially between 4.0% and 9.0% by weight, relative to the total weight of the composition.

### Surfactants

A composition according to the invention may comprise emulsifying surfactants, preferably that are nonionic, different from the polyglycerol esters defined above.

Of course, the emulsifying surfactant(s) optionally present in a composition according to the invention is (are) chosen so as not to impair the properties of the composition, in particular in terms of stability.

Examples of emulsifying surfactants that may be mentioned include alkyl esters or ethers of sorbitan, of polyols or of sugars, different from the polyglycerols.

Examples of polyol alkyl esters that may be mentioned include polyethylene glycol esters, for instance PEG-30 dipolyhydroxystearate, such as the product sold under the name Arlacel^{®} P135 by the company ICI.

Examples of glycerol and/or sorbitan esters that may be mentioned include sorbitan isostearate, such as the product sold under the name Arlacel^{®} 987 by the company ICI or sorbitan glyceryl isostearate, such as the product sold under the name Arlacel^{®} 986 by the company ICI, and mixtures thereof.

Examples of emulsifying surfactants that may also be mentioned include silicone surfactants, such as dimethicone copolyols or silicone elastomers.

Examples of dimethicone copolyols that may be mentioned include the one with the INCI name Dimethicone (and) PEG/PPG-18/18 Dimethicone sold under the brand X-22-6711D^{®} by the company SHIN ETSU, the mixture of cyclomethicone and dimethicone copolyol, sold under the name DC5225^{®} C by the company Dow Corning, and alkyl dimethicone copolyols such as the lauryl methicone copolyol sold under the name DC5200 Formulation Aid by the company Dow Corning; cetyl dimethicone copolyol, for instance cetyl PEG/PPG-10/1 Dimethicone such as the product sold under the name Abil EM^{®} 90 by the company EVONIK GOLDSCHMIDT.

According to a preferred embodiment, a composition according to the invention comprises at least one emulsifying surfactant chosen from glycerol esters of a C₈-C₂₄, in particular C₁₂-C₂₂, fatty acid such as glycerol ester of stearic acid (CTFA name: Glyceryl stearate).

A composition according to the invention may comprise between 0.01% and 2.0% by weight of emulsifying surfactant, different from the polyglycerol esters defined above, in particular a glycerol ester of stearic acid, preferably between 0.05% and 1.5% by weight, more preferentially between 0.1% and 1.0% by weight, relative to the total weight of the composition.

### Fillers

Advantageously, a composition according to the invention may also comprise one or more fillers, in particular chosen from those conventionally used in care and/or makeup compositions.

For the purposes of the present invention, the term "fillers" is understood to mean colorless or white, mineral or organic, natural or synthetic solid particles of any form, which are in a form that is insoluble and dispersed in the medium of the composition.

Of course, these fillers are used in appropriate contents and under appropriate conditions so as not to be detrimental to the properties of the composition.

These fillers make it possible to give the composition containing them softness, a matt effect and uniformity of the makeup result. In addition these fillers advantageously make it possible to combat various attacking factors such as sebum or sweat.

As illustrations of these fillers, mention may be made of talc, mica, silica, kaolin, poly-β-alanine powder and polyethylene powder, powders of tetrafluoroethylene polymers (Teflon^{®}), lauroyl lysine, starch, boron nitride, hollow polymer microspheres such as those of polyvinylidene chloride/acrylonitrile, for instance Expancel^{®} (Nobel Industrie), acrylic acid copolymer microspheres, silicone resin microbeads (for example Tospearls^{®} from Toshiba), polyorganosiloxane elastomer particles, precipitated calcium carbonate, magnesium carbonate, magnesium hydrogen carbonate, hydroxyapatite, barium sulfate, aluminum oxides, polyurethane powders, composite fillers, hollow silica microspheres, and glass or ceramic microcapsules. Use may also be made of particles that are in the form of hollow sphere portions, as described in the patent applications JP-2003 128 788 and JP-2000 191 789.

Preferably, a composition in accordance with the invention comprises at least one filler, in particular chosen from lauroyl lysine, talc, calcium carbonate, and mixtures thereof.

In particular, such fillers may be present in a composition according to the invention in a content of between 0.5% and 10% by weight, especially between 1.0% and 7.0% by weight, in particular between 1.5% and 5.0% by weight, relative to the total weight of the composition.

### Colorants

A composition according to the invention may also comprise at least one particulate or non-particulate, water-soluble or water-insoluble colorant, preferably in a proportion of at least 0.01% by weight relative to the total weight of the composition.

For obvious reasons, this amount is liable to vary significantly with regard to the intensity of the desired color effect and of the color intensity afforded by the colorants under consideration, and its adjustment clearly falls within the competence of a person skilled in the art.

A composition according to the invention may comprise from 0.01% to 25% by weight, especially from 0.1% to 25% by weight, in particular from 1.0% to 20% by weight and preferably from 2.5% to 15% by weight of colorants relative to the total weight of said composition.

As stated above, the colorants that are suitable for use in the invention may be water-soluble, but may also be liposoluble.

For the purposes of the invention, the term "water-soluble colorant" means any natural or synthetic, generally organic compound, which is soluble in an aqueous phase or water-miscible solvents and which is capable of imparting color.

As water-soluble dyes that are suitable for use in the invention, mention may be made especially of synthetic or natural water-soluble dyes, for instance FDC Red 4, DC Red 6, DC Red 22, DC Red 28, DC Red 30, DC Red 33, DC Orange 4, DC Yellow 5, DC Yellow 6, DC Yellow 8, FDC Green 3, DC Green 5, FDC Blue 1, betanin (beetroot), carmine, copper chlorophyllin, methylene blue, anthocyanins (enocianin, black carrot, hibiscus and elder), caramel and riboflavin.

The water-soluble dyes are, for example, beetroot juice and caramel.

For the purposes of the invention, the term "liposoluble colorant" means any natural or synthetic, generally organic compound, which is soluble in an oily phase or in solvents that are miscible with a fatty substance, and which is capable of imparting color.

As liposoluble dyes that are suitable for use in the invention, mention may be made especially of synthetic or natural liposoluble dyes, for instance DC Red 17, DC Red 21, DC Red 27, DC Green 6, DC Yellow 11, DC Violet 2, DC Orange 5, Sudan red, carotenes (β-carotene, lycopene), xanthophylls (capsanthin, capsorubin, lutein), palm oil, Sudan brown, quinoline yellow, annatto and curcumin.

The coloring particulate materials may be present in a proportion of from 0.01% to 25% by weight relative to the total weight of the composition containing them.

They may especially be pigments, nacres and/or particles with metallic tints.

The term "pigments" should be understood as meaning white or colored, mineral or organic particles that are insoluble in an aqueous solution, which are intended to color and/or opacify the composition containing them.

A composition according to the invention may comprise from 0.01% to 25% by weight, especially from 0.1% to 25% by weight, in particular from 1.0% to 25% by weight and preferably from 2.5% to 15% by weight of pigments relative to the total weight of said composition.

Preferably, when the composition according to the invention is a makeup composition, it may comprise at least 2.5% by weight, preferably at least 10% by weight and more preferentially at least 15% by weight, of pigments relative to the total weight of said composition.

The pigments may be white or colored, and mineral and/or organic.

As mineral pigments which may be used in the invention, mention may be made of titanium oxide, titanium dioxide, zirconium oxide, zirconium dioxide, cerium oxide or cerium dioxide and also zinc oxide, iron oxide or chromium oxide, ferric blue, manganese violet, ultramarine blue and chromium hydrate, and mixtures thereof.

They may also be pigments having a structure that may be, for example, of sericite/brown iron oxide/titanium dioxide/silica type. Such a pigment is sold, for example, under the reference Coverleaf NS or JS by the company Chemicals and Catalysts, and has a contrast ratio in the region of 30.

They may also be pigments having a structure that may be, for example, of silica microsphere type containing iron oxide. An example of a pigment having this structure is that sold by the company Miyoshi under the reference PC Ball PC-LL-100 P, this pigment being constituted of silica microspheres containing yellow iron oxide.

Advantageously, the pigments in accordance with the invention are iron oxides and/or titanium dioxides.

The term"nacres" should be understood as meaning iridescent or non-iridescent colored particles of any shape, notably produced by certain molluscs in their shell or alternatively synthesized, which have a color effect via optical interference.

A composition according to the invention may comprise from 0% to 15% by weight of nacres relative to the total weight of said composition.

The nacres may be chosen from nacreous pigments such as titanium mica coated with an iron oxide, titanium mica coated with bismuth oxychloride, titanium mica coated with chromium oxide, titanium mica coated with an organic dye and also nacreous pigments based on bismuth oxychloride. They may also be mica particles, at the surface of which are superposed at least two successive layers of metal oxides and/or of organic colorants.

Examples of nacres that may also be mentioned include natural mica coated with titanium oxide, with iron oxide, with natural pigment or with bismuth oxychloride.

Among the nacres available on the market, mention may be made of the nacres Timica, Flamenco and Duochrome (based on mica) sold by the company Engelhard, the Timiron nacres sold by the company Merck, the Prestige mica-based nacres sold by the company Eckart, and the Sunshine synthetic mica-based nacres sold by the company Sun Chemical.

The nacres may more particularly have a yellow, pink, red, bronze, orange, brown, gold and/or coppery color or tint.

Advantageously, the nacres in accordance with the invention are micas covered with titanium dioxide or with iron oxide, and also bismuth oxychloride.

For the purposes of the present invention, the term "particles with a metallic tint" means any compound whose nature, size, structure and surface finish allow it to reflect incident light, in particular in a non-iridescent manner.

The particles with a metallic tint that can be used in the invention are in particular chosen from:
- particles of at least one metal and/or of at least one metal derivative;
- particles comprising a monomaterial or multimaterial organic or inorganic substrate, at least partially coated with at least one layer with a metallic tint comprising at least one metal and/or at least one metal derivative; and
- mixtures of said particles.

Among the metals that may be present in said particles, mention may for example be made of Ag, Au, Cu, Al, Ni, Sn, Mg, Cr, Mo, Ti, Zr, Pt, Va, Rb, W, Zn, Ge, Te, Se and mixtures or alloys thereof. Ag, Au, Cu, Al, Zn, Ni, Mo and Cr, and mixtures or alloys thereof (for example bronzes and brasses), are preferred metals.

The term "metal derivatives" denotes compounds derived from metals, in particular oxides, fluorides, chlorides and sulfides.

Illustrations of these particles that may be mentioned include aluminum particles, such as those sold under the names Starbrite 1200 EAC^{®} by the company Siberline and Metalure^{®} by the company Eckart and glass particles coated with a metallic layer, especially those described in documents JP-A-09188830, JP-A-10158450, JP-A-10158541, JP-A-07258460 and JP-A-05017710.

### Hydrophobic treatment of the colorants

The pulverulent colorants as described previously may be totally or partially surface treated, with a hydrophobic agent, to make them more compatible with the oily phase of the composition of the invention, especially so that they have good wettability with oils. Thus, these treated pigments are well dispersed in the oily phase.

Hydrophobically treated pigments are described especially in document EP-A-1 086 683. The hydrophobic-treatment agent may be chosen from silicones such as methicones, dimethicones and perfluoroalkylsilanes; fatty acids, such as stearic acid; metal soaps, such as aluminum dimyristate, the aluminum salt of hydrogenated tallow glutamate; perfluoroalkyl phosphates; polyhexafluoropropylene oxides; perfluoropolyethers; amino acids; N-acylamino acids or salts thereof; lecithin, isopropyl triisostearyl titanate, isostearyl sebacate, and mixtures thereof.

The term "alkyl" mentioned in the compounds cited above especially denotes an alkyl group having from 1 to 30 carbon atoms and preferably having from 5 to 16 carbon atoms.

### Active agents

According to one preferred embodiment, a composition according to the invention also comprises at least one additional cosmetic active agent.

In particular, the additional cosmetic active agent may be at least one hydrophilic active agent and/or one lipophilic active agent.

As cosmetic active agents, examples that may be mentioned include moisturizers, depigmenting agents, desquamating agents, humectants, antiaging agents; mattifying agents, cicatrizing agents, antibacterial agents, vitamins and derivatives thereof, antioxidant compounds, sunscreens and mixtures thereof.

The additional active agent(s) may notably be chosen from:
- vitamins and derivatives thereof, in particular esters thereof;
- humectants, for instance urea, hydroxyureas, glycerol, polyglycerols, glyceryl glucoside, diglyceryl glucoside, polyglyceryl glucosides and xylityl glucoside;
- antioxidant compounds;
- antiaging active agents, such as hyaluronic acid compounds and salicylic acid compounds;
- sunscreens; and
- mixtures thereof.

Such active agents may be present in a composition according to the invention in a content ranging from 0.05% to 5.0% by weight, preferably from 0.05% to 1.5% by weight, relative to the total weight of the composition.

### Intended use of the composition

A composition according to the invention may be in the form of a cosmetic composition for caring for and/or making up keratin materials, preferably a cosmetic composition for caring for keratin materials, in particular of the body or of the face, preferably of the face.

These compositions may constitute cleansing, protective, treating or care creams for the face, the hands or the body, for example day creams, night creams, makeup creams, foundation creams or antisun creams.

According to one embodiment, a composition according to the invention is in the form of a composition for caring for keratin materials, in particular the skin of the body or the face, preferably of the face.

In particular, a composition of the invention may be in the form of an antiaging care composition for the skin of the body or the face, in particular of the face.

According to another embodiment, a composition of the invention may be in the form of a composition for making up keratin materials, in particular of the body or of the face, preferably of the face.

Thus, according to a submode of this embodiment, a composition of the invention may be in the form of a makeup base composition for making up. A composition of the invention may in particular be in the form of a foundation or a tinted cream.

Such compositions are in particular prepared according to the general knowledge of those skilled in the art.

Thus, the invention also relates to the use of a composition according to the invention for caring for and/or making up keratin materials, preferably for caring for keratin materials, in particular the skin of the body and/or of the face.

The invention also relates to a cosmetic process for making up and/or caring for keratin materials, in particular the skin, comprising at least one step of applying a composition as defined previously to said keratin materials.

Preferably, the invention also relates to a cosmetic process for caring for keratin materials, in particular the skin, comprising at least one step of applying a composition as defined above to said keratin materials.

The cosmetic processes for making up and/or caring for keratin materials, in particular the skin, are non-therapeutic.

In particular, a composition according to the invention may be used for combating the signs of skin aging.

Thus, the present application also relates to the use of a composition according to the invention for combating the signs of skin aging.

The composition may be applied to the skin by hand or using an applicator.

The expressions "between... and...", "comprises from ... to...", "formed from ... to..." and "ranging from... to..." should be understood as being inclusive of the limits, unless otherwise specified.

The invention is illustrated in greater detail by the examples presented below. Unless otherwise indicated, the amounts shown are expressed as weight percentages.

### Example

### Measurement and evaluation methods

### Stability measurement

A first method of evaluating the stability is carried out by observation of the composition over time, compared to a reference.

The instability may be detected by evaluating salting out, creaming, coalescence, formation of a film at the surface, marbling, etc.

Variations in texture, appearance, color and odor may also be evaluated.

In particular, the compositions are observed after storage for 2 months at a temperature of around 45°C.

Another method of evaluating the stability may be carried out by measuring the viscosity.

The viscosity of the product is measured with a Rheomat 35 RM 180 viscometer according to the CID-012-02 method. The measurement of the torque needed to overcome the resistance of the fluid whose viscosity it is desired to determine is carried out using a submerged element (spindle or measuring body) rotating at a chosen and constant speed (5 rpm). The measurement is performed with a product to be analyzed/cup/measuring body assembly at 25°C.

The spindle used to carry out the measurements is chosen depending on the viscosity of the product to be analyzed:
- M1: lotion;
- M2: fluid milk;
- M3: cream, thick milk;
- M4: thick cream;
- Penetrometer: textures of balm type (forces exerted in grams in the product).

The volume of substance introduced into the cup of the spindle is 25 ml. The support for the system and the measuring body are placed in the machine for the measurement.

It is measured at 25°C on a Rheomat. Several spindles may be used depending on the viscosity of the fluid.

The results are expressed in deviation units (DU). The standard deviation is ± 5 DU for viscosity values of less than or equal to 50 DU and ± 7 DU for viscosity values of greater than 50.

Yet another method for evaluating the stability consists in observing the formulas with a microscope in non-polarized light at magnifications of ×100 and if need be ×400.

This makes it possible to evaluate the fineness of the drops and/or the absence of clusters and/or the presence of crystals.

Lastly, the stability of the composition may be evaluated by examining the defects of the composition after several temperature change cycles.

Each cycle is constituted of the following steps:
a) maintaining the composition at 20°C for 6 hours;
b) decreasing the temperature to -20°C over a period of 6 hours;
c) maintaining the composition at -20°C for 6 hours; and
d) increasing the temperature to 20°C over a period of 6 hours.

Ten cycles are carried out and the defects of the composition are evaluated between each cycle.

The appearance of defects between the first and fifth cycles inclusive indicates significant instability of the composition.

### Evaluation of the sensory properties

The sensory properties of a composition may be evaluated by a panel of 30 people who are authorized and trained, having applied the composition daily for 10 days.

In particular, the tackiness, greasy effect and glidance during application and after application on the skin are determined.

### Example 1

The antiaging care compositions for the face, in the form of water-in-oil emulsions, 1 and 2 according to the invention, are prepared in the weight proportions as described in detail in table 1 below. The values are expressed as weight percentages relative to the total weight of the composition.

**Table 1**

| **Phase** | **Compounds (INCI Name)** | **Formula 1 according to the invention** | **Formula 2 according to the invention** |
|---|---|---|---|
| A | Glyceryl Stearate *(TEGIN M PELLETS*^{®} *from Evonik)* | 0.5 | 0.5 |
| | Dicaprylyl Ether (*Cetiol*^{®} *OE from BASF)* | 12.0 | 12.0 |
| | Undecane (and) Tridecane (*Cetiol*^{®} *UT from BASF)* | 7.0 | 3.0 |
| | C₁₅-C₁₉ Alkane *(Emogreen*^{®} *L15 from Evonik)* | - | 4.0 |
| | Polyglyceryl-4 Diisostearate / Polyhydroxystearate / Sebacate (and) Caprylic/Capric Triglyceride (and) Polyglyceryl-3 Oleate (and) Diisostearoyl Polyglyceryl-3 Dimer Dilinoleate (*Isolan*^{®} *17 MB from Evonik*) | 4.0 | 4.0 |
| | Polyglyceryl-4 isostearate (*Isolan*^{®} *GI34 from Evonik)* | 2.0 | 2.0 |
| B | Trihydroxystearin *(Thixcin*^{®} *R from Elémentis)* | 0.5 | 0.5 |
| C | Water | qs 100 | qs 100 |
| | Magnesium Sulfate *(RONACARE*^{®} *MAGNESIUM SULFATE from Merck)* | 2.0 | 2.0 |
| | Phytic acid *(PHYTIC ACID 50% SOLUTION*^{®} *from Tsuno)* | - | 0.15 |
| | Propanediol *(1,3-PROPANEDIOL from ZHANGJIAGANG GLORY CHEMICAL)* | - | 3.0 |
| | Glycerin *(GLYCERINE 4811 RSPO MB from OLEON)* | 5.0 | 5.0 |
| | Salicylic Acid *(Salicylic Acid from JQC)* | 0.2 | 0.2 |
| | Arginine *(L-ARGININE C GRADE from AJINOMOTO)* | 0.2 | 0.2 |
| | Potassium Sorbate *(RONACARE*^{®} *POTASSIUM SORBATE from Merck)* | 0.15 | 0.15 |
| D | Lauroyl Lysine *(AMIHOPE*^{®} *LL from AJINOMOTO)* | 2.0 | 2.0 |
| E | Ethanol *(ETHANOL TSDA COSMOS ORGANIC CT from Earth Oil)* | 5.0 | 5.0 |

### Protocol for preparing the compositions

The compositions are prepared according to the protocol described in detail below, in a Minilab reactor.

Place phase A in the tank and heat at 75°C until the glyceryl stearate has dissolved under paddle stirring, then reduce the temperature to 53°C.

At the same time, weigh phase C and heat in a deflocculator at 50°C until completely homogenized.

Separate phase B into two, by withdrawing 300 g into a beaker. Add B to the tank at a temperature of between 50°C and 55°C then stir for 10 minutes with the aid of paddles at 50 rpm and a rotor at 4000 rpm.

Add the first portion of phase B to the tank at 50-55°C via the inlet cone over 5 minutes then homogenize under vacuum at -0.5 bar for around 3 minutes with the aid of paddles at 70 rpm and a rotor at 4000 rpm.

Start the cooling in a water bath at 20°C and leave stirring under vacuum at -0.5 bar with the aid of paddles at 40 rpm and a rotor at 2000 rpm.

When the temperature is below 30°C, incorporate the second portion of phase B from the beaker via the inlet cone dropwise over 15 minutes with stirring under vacuum at -0.5 bar with the aid of paddles at 70 rpm and a rotor at 4000 rpm.

Next incorporate the filler via the top of the tank over around 10 seconds with stirring with the aid of paddles at 60 rpm then scraping of the paddles followed by 5 minutes of stirring under vacuum at -0.5 bar with the aid of paddles at 70 rpm and a rotor at 4000 rpm. Incorporate the alcohol phase via the inlet cone over around 5 minutes followed by 5 minutes of homogenization under vacuum at -0.5 bar with the aid of paddles at 70 rpm and a rotor at 4000 rpm.

### Comparison and results

The sensory properties of formulas 1 and 2 according to the invention were evaluated by the panel, as described in detail above, and compared to those of the commercial compositions listed in table 2 below.

**Table 2**

| **Composition** | **Trade name** | **Type** |
|---|---|---|
| Formula 3 outside the invention | Lancôme Renergie Multi Lift Ultra | Oil-in-Water Emulsion |
| Formula 4 outside the invention | Lancôme ABC day cream | Oil-in-Water Emulsion |
| Formula 5 outside the invention | OAP revitalift | Oil-in-Water Emulsion |
| Formula 6 outside the invention | SKII RNA power radical new age | Oil-in-Water Emulsion |
| Formula 7 outside the invention | Estee Lauder revitalizing supreme | Oil-in-Water Emulsion |

Formulas 3 to 7 outside the invention differ from the compositions according to the invention due to their presentation form, since these are oil-in-water emulsions.

Furthermore, these formulas do not comprise any polyglycerol ester in accordance with the invention and are also free of glyceryl hydroxy stearate.

### Results

Formula 1 according to the invention makes it possible to obtain long-lasting moisturization of the skin, a radiant and regenerated complexion, masking fine lines and signs of fatigue. The skin appears younger and healthy.

Formula 1 according to the invention is lighter, and provides a better antiaging benefit, compared to formula 3 outside the invention.

Formula 1 according to the invention has better performance in terms of greasy effect on application, tackiness an application and greasy finish on the skin, compared to formula 4 outside the invention. Furthermore, formula 1 according to the invention has lower glidance on application than the formula 5 outside the invention.

Formula 2 according to the invention is not very greasy and not very tacky. In particular, it is less tacky on application than formula 6 outside the invention and less greasy on application than formula 7 outside the invention.

Furthermore, formulas 1 and 2 according to the invention are stable.

### Example 2

The antiaging care compositions for the face, in the form of water-in-oil emulsions, 8 to 12 according to the invention, are prepared in the weight proportions as described in detail in table 3 below. The values are expressed as weight percentages relative to the total weight of the composition.

**Table 3**

| **Phase** | **Compounds (INCI Name)** | **Formula 8 (invention)** | **Formula 9 (invention)** | **Formula 10 (invention)** | **Formula 11 (invention)** | **Formula 12 (invention)** |
|---|---|---|---|---|---|---|
| A | Glyceryl Stearate *(TEGIN M PELLETS*^{®} *from Evonik*) | 0.5 | - | - | - | - |
| | Dicaprylyl Ether *(Cetiol*^{®} *OE from BASF)* | 12.0 | - | - | 19.0 | - |
| | Undecane (and) Tridecane *(Cetiol*^{®} *UT from BASF)* | 7.0 | - | - | - | - |
| | C15-C19 Alkane *(Emogreen*^{®} *L15 from Evonik*) | - | 19.0 | - | - | - |
| | Caprylic / capric triglyceride *(DUB*^{®} *MCT 7030 from Stearinerie dubois)* | - | - | 19.0 | - | 19.0 |
| | Polyglyceryl-4 isostearate (*Isolan*^{®} *GI34 from Evonik)* | 2.0 | 2.0 | 2.0 | 2.0 | 2.0 |
| | Polyglyceryl-4 Diisostearate / Polyhydroxystearate / Sebacate (and) Caprylic/Capric Triglyceride (and) Polyglyceryl-3 Oleate (and) Diisostearoyl Polyglyceryl-3 Dimer Dilinoleate (*Isolan*^{®} *17 MB from Evonik*) | 4.0 | 4.0 | 4.0 | 4.0 | 4.0 |
| B | Trihydroxystearin *(Thixcin*^{®} *R from Elémentis)* | 0.5 | 0.5 | 0.5 | 0.5 | 0.5 |
| C | Water | qs 100 | qs 100 | qs 100 | qs 100 | qs 100 |
| | Magnesium Sulfate *(RONACARE*^{®} *MAGNESIUM SULFATE from Merck)* | 2.0 | 2.0 | 2.0 | 2.0 | 2.0 |
| | Glycerin *(GLYCERINE 4811 RSPO MB from OLEON)* | 5.0 | 5.0 | 5.0 | 5.0 | 5.0 |
| | Salicylic Acid *(Salycilic Acid from JQC)* | 0.2 | 0.2 | 0.2 | 0.2 | 0.2 |
| | Arginine *(L-ARGININE C GRADE from AJINOMOTO)* | 0.2 | 0.2 | 0.2 | 0.2 | 0.2 |
| | Sodium benzoate *(SODIUM BENZOATE POWDER from Wuhan youji)* | 0.15 | 0.15 | 0.15 | 0.15 | 0.15 |
| D | Lauroyl Lysine *(AMIHOPE*^{®} *LL from AJINOMOTO)* | 2.0 | 2.0 | 2.0 | 2.0 | - |
| D | Talc *(IMERCARE*^{®} *PHARMA from Imerys)* | - | - | - | - | 2.0 |

### Results

Formulas 8 to 12, according to the invention, are stable and have good sensory properties. In particular, formula 9 has good glidance and affords a soft finish after application.

### Example 3

The antiaging care composition for the face, in the form of a water-in-oil emulsion, 13 outside the invention, in particular of which the polyglycerol ester of at least one poly(hydroxystearic acid) and carboxylic acids/polyglycerol fatty acid ester weight ratio is equal to 0.7, is prepared in the weight proportions as described in detail in table 4 below. The values are expressed as weight percentages relative to the total weight of the composition.

**Table 4**

| **Phase** | **Compounds (INCI Name)** | **Formula 13 Outside invention** |
|---|---|---|
| A | Polyglyceryl-4 Diisostearate / Polyhydroxystearate / Sebacate (and) Caprylic/Capric Triglyceride (and) Polyglyceryl-3 Oleate (and) Diisostearoyl Polyglyceryl-3 Dimer Dilinoleate (*Isolan*^{®} *17 MB from Evonik*) | 5.0 |
| | Polyglyceryl-4 isostearate (*Isolan*^{®} *GI34 from Evonik)* | 5.0 |
| | Dicaprylyl Ether *(Cetiol^{®} OE from BASF)* | 11.0 |
| | Undecane (and) Tridecane *(Cetiol*^{®} *UT from BASF)* | 6.0 |
| B | Trihydroxystearin *(Thixcin*^{®} *R from Elémentis)* | 0.5 |
| C | Water | qs 100 |
| | Magnesium Sulfate *(RONACARE*^{®} *MAGNESIUM SULFATE from Merck)* | 2.0 |
| | Glycerin *(GLYCERINE 4811 RSPO MB from OLEON)* | 5.0 |
| | Pentylène glycol *(A-LEEN*^{®} *5 from Minasolve)* | 4.0 |
| | Benzyl alcohol *(162053 BENZYL ALCOHOL DD from Symrise)* | 0.7 |
| | Potassium Sorbate *(RONACARE*^{®} *POTASSIUM SORBATE from Merck)* | 0.15 |
| D | Lauroyl Lysine *(AMIHOPE*^{®} *LL from AJINOMOTO)* | 2.0 |

### Result

Formula 13 is not stable and exhibits phase separation after cyclical oven treatment.

### Example 4

The antiaging care compositions for the face, in the form of water-in-oil emulsions, 14 to 16 outside the invention, in particular comprising no polyglycerol fatty acid ester as defined according to the invention, are prepared in the weight proportions as described in detail in table 5 below. The values are expressed as weight percentages relative to the total weight of the composition.

**Table 5**

| **Phase** | **Compounds (INCI Name)** | **Formula 14 Outside invention** | **Formula 15 Outside invention** | **Formula 16 Outside invention** |
|---|---|---|---|---|
| A | Glyceryl Stearate *(TEGIN M PELLETS*^{®} *from Evonik)* | - | 2.0 | 1.0 |
| | Polyglyceryl-4 Diisostearate / Polyhydroxystearate / Sebacate (and) Caprylic/Capric Triglyceride (and) Polyglyceryl-3 Oleate (and) Diisostearoyl Polyglyceryl-3 Dimer Dilinoleate (*Isolan*^{®} *17 MB from Evonik)* | 4.0 | 4.0 | 4.0 |
| | Dicaprylyl Ether *(Cetiol*^{®} *OE from BASF)* | - | - | 19.0 |
| | Sorbitan trioleate *(NIKKOL*^{®} *SO 30V from Nippon)* | 2.0 | - | - |
| | Dodecane *(PARAFOL*^{®} *12-97 from Sasol)* | 19.0 | 19.0 | - |
| B | Trihvdroxvstearin *(Thixcin*^{®} *R from Elémentis)* | 0.5 | 0.5 | 0.5 |
| C | Water | qs 100 | qs 100 | qs 100 |
| | Magnesium Sulfate *(RONACARE*^{®} *MAGNESIUM SULFATE from Merck)* | 2.0 | 2.0 | 2.0 |
| | Glycerin *(GLYCERINE 4811 RSPO MB from OLEON)* | 5.0 | 5.0 | 5.0 |
| | Salicylic Acid *(Salycilic Acid from JQC)* | 0.2 | 0.2 | 0.2 |
| | Arginine *(L-ARGININE C GRADE from AJINOMOTO)* | 0.2 | 0.2 | 0.2 |
| | Sodium benzoate *(SODIUM BENZOATE POWDER from Wuhan youji)* | 0.15 | 0.15 | 0.15 |
| D | Lauroyl Lysine *(AMIHOPE*^{®} *LL from AJINOMOTO)* | 2.0 | 2.0 | 2.0 |

### Results

Formulas 14 to 16 do not exhibit satisfactory stability, in particular evaluated by a cyclical study. Also, after storage for 2 months at 45°C, phase separation of the compositions is observed.

### Example 5

The antiaging care compositions for the face, in the form of water-in-oil emulsions, 17 to 20 outside the invention, in particular comprising neither a polyglycerol fatty acid ester nor glycerol hydroxystearate as defined according to the invention, are prepared in the weight proportions as described in detail in table 6 below.

The values are expressed as weight percentages relative to the total weight of the composition.

**Table 6**

| **Phase** | **Compounds (INCI Name)** | **Formula 17 Outside invention** | **Formula 18 Outside invention** | **Formula 19 Outside invention** | **Formula 20 Outside invention** |
|---|---|---|---|---|---|
| A | Polyglyceryl-4 Diisostearate / Polyhydroxystearate / Sebacate (and) Caprylic/Capric Triglyceride (and) Polyglyceryl-3 Oleate (and) Diisostearoyl Polyglyceryl-3 Dimer Dilinoleate (*Isolan*^{®} *17 MB from Evonik*) | 3.0 | 3.0 | 3.0 | 3.0 |
| | Octyldodecanol *(Eutanol*^{®} *G from BASF)* | 19.0 | - | - | - |
| | Caprylic / capric triglyceride *(DUB*^{®} *MCT 7030 from Stearinerie dubois)* | - | 19.0 | - | - |
| | Hexyl decyl laurate (and) hexyldecanol *(Cetiol*^{®} *PGL from BASF)* | - | - | 19.0 | - |
| | Dicaprylyl carbonate *(Cetiol*^{®} *CC from BASF)* | - | - | - | 19.0 |
| B | Water | qs 100 | qs 100 | qs 100 | qs 100 |
| | Magnesium Sulfate *(RONACAR*^{®}*E MAGNESIUM SULFATE from Merck)* | 2.0 | 2.0 | 2.0 | 2.0 |
| | Propylene glycol (1,2-*PROPYLENEGLYCOL CARE from BASF)* | 5.0 | 5.0 | 5.0 | 5.0 |
| | Glycerin *(GLYCERINE 4811 RSPO MB from OLEON)* | 5.0 | 5.0 | 5.0 | 5.0 |

### Results

Formulas 17 to 20, not in accordance with the invention, are stable but do not exhibit satisfactory sensory properties. In particular, formulas 18 to 20 leave a greasy and shiny finish after application on the skin.

Moreover, formula 18 gives rise to the formation of plaques and composition 20 has a film-forming appearance. Furthermore, these compositions are not able to be stored in a satisfactory manner.

## Claims

1. A composition, especially a cosmetic composition, in particular for making up and/or caring for keratin materials, comprising:
- at least one continuous fatty phase;
- at least one aqueous phase at a concentration of at least 50.0% by weight, relative to the total weight of the composition, and dispersed in said fatty phase;
- at least one polyglycerol ester of at least one poly(hydroxystearic acid) and carboxylic acids, derived from the esterification between at least one polyglycerol and (i) at least one poly(hydroxystearic acid); (ii) at least one di- and/or tricarboxylic acid; and (iii) at least one saturated or unsaturated, linear or branched fatty acid having from 6 to 22 carbon atoms;
- at least one ester of polyglycerol comprising from 4 to 15 glycerol units and of at least one linear or branched, saturated or unsaturated fatty acid comprising from 6 to 24 carbon atoms; and
- glyceryl trihydroxystearate;
the polyglycerol ester of at least one poly(hydroxystearic acid) and carboxylic acids/polyglycerol ester of at least one fatty acid weight ratio being strictly greater than 0.7, preferably greater than 1.0.

2. The composition as claimed in claim 1, **characterized in that** the polyglycerol ester of at least one poly(hydroxystearic acid) and carboxylic acids is derived from the esterification between a mixture of polyglycerol and (i) of poly(hydroxystearic acid) with 4 polyglycerol units; (ii) of linear or branched, aliphatic dicarboxylic acids having 8 to 12 carbon atoms; and (iii) of saturated or unsaturated, linear or branched fatty acids having from 16 to 20 carbon atoms, and preferably is polyglyceryl-4 diisostearate/polyhydroxystearate/sebacate.

3. The composition as claimed in claim 1 or 2, comprising between 0.5% and 5.0% by weight, preferably between 1.0% and 4.0% by weight, more preferentially between 1.5% and 3.5% by weight, of at least one polyglycerol ester of at least one poly(hydroxystearic acid) and carboxylic acids, relative to the total weight of the composition.

4. The composition as claimed in any one of the preceding claims, **characterized in that** the polyglycerol fatty acid ester is chosen from esters derived from the esterification reaction between a polyglycerol comprising from 4 to 5 glycerol units, in particular comprising 4 glycerol units, and at least one aliphatic carboxylic acid comprising an alkyl chain having from 16 to 20 carbon atoms, such as a stearyl and/or isostearyl chain.

5. The composition as claimed in any one of the preceding claims, comprising between 0.1% and 5.0% by weight, preferably between 0.5% and 4.0% by weight, more preferentially between 1.0% and 4.0% by weight, of at least one polyglycerol fatty acid ester, relative to the total weight of the composition.

6. The composition as claimed in any one of the preceding claims, comprising between 0.01% and 2.0% by weight, in particular between 0.1% and 1.5% by weight, preferably between 0.1% and 1% by weight, more preferentially between 0.2% and 0.8% by weight, of glyceryl trihydroxystearate, relative to the total weight of the composition.

7. The composition as claimed in any one of the preceding claims, comprising between 5.0% and 50% by weight of fatty phase, in particular between 10% and 30% by weight, and preferably between 15% and 25% by weight, relative to the total weight of the composition.

8. The composition as claimed in any one of the preceding claims, comprising less than 2.0% by weight of silicone oil(s), in particular less than 1.0% by weight of silicone oil(s), preferably less than 0.5% by weight of silicone oil(s), relative to the total weight of the composition, and more preferentially being free of silicone oil(s).

9. The composition as claimed in any one of the preceding claims, comprising at least one hydrocarbon-based oil chosen from volatile linear alkanes comprising from 11 to 13 carbon atoms, in particular an undecane-tridecane mixture, and linear and/or branched C₁₅-C₁₉ alkanes, in particular a mixture of linear or branched C₁₅-C₁₉ alkanes.

10. The composition as claimed in any one of the preceding claims, comprising at least one nonvolatile hydrocarbon-based oil chosen from synthetic ethers having from 10 to 40 carbon atoms, such as dicaprylyl ether, carbonates, such as dicaprylyl carbonate, fatty acid triglycerides, in particular that are saturated, such as caprylic/capric triglyceride, and mixtures thereof.

11. The composition as claimed in any one of the preceding claims, comprising from 50% to 90% by weight of aqueous phase, preferably from 60% to 85% by weight, and more preferentially from 65% to 75% by weight, relative to the total weight of said composition.

12. The composition as claimed in any one of the preceding claims, further comprising at least one preservative, in particular a mixture of preservatives comprising at least one alcohol, preferably ethanol, at least one glycol, preferably pentylene glycol, salicylic acid, potassium sorbate and phytic acid.

13. The composition as claimed in any one of the preceding claims, further comprising at least one emulsifying surfactant different from the polyglycerol esters, preferably chosen from glycerol esters of C₈-C₂₄ fatty acids, in particular C₁₂-C₂₂ fatty acids, such as the glycerol ester of stearic acid.

14. The composition as claimed in any one of the preceding claims, further comprising at least one filler, in particular chosen from lauroyl lysine, talc, calcium carbonate, and mixtures thereof.

15. The composition as claimed in any one of the preceding claims, further comprising at least one polyol, in particular chosen from glycerin, propanediol, and mixtures thereof.

16. The composition as claimed in any one of the preceding claims, **characterized in that** it has a viscosity ranging from 0.1 to 10 Pa.s, preferably from 0.5 to 8.0 Pa.s, more preferentially from 2.5 to 7.0 Pa.s.

17. A cosmetic process for caring for keratin materials, in particular the skin, comprising at least one step of applying to said keratin materials a composition as defined in any one of claims 1 to 16.

## Patentansprüche

1. Zusammensetzung, insbesondere eine kosmetische Zusammensetzung, insbesondere zum Schminken und/oder Pflegen von Keratinmaterialien, umfassend
- mindestens eine kontinuierliche Fettphase;
- mindestens eine wässrige Phase in einer Konzentration von mindestens 50,0 Gew.-%, bezogen auf das Gesamtgewicht der Zusammensetzung, die in der Fettphase dispergiert ist;
- mindestens einen Polyglycerinester aus mindestens einer Poly(hydroxystearinsäure) und Carbonsäuren, der aus der Veresterung zwischen mindestens einem Polyglycerin und (i) mindestens einer Poly(hydroxystearinsäure); (ii) mindestens einer Di- und/oder Tricarbonsäure; und (iii) mindestens einer gesättigten oder ungesättigten, linearen oder verzweigten Fettsäure mit 6 bis 22 Kohlenstoffatomen abgeleitet ist;
- mindestens einen Ester von Polyglycerin mit 4 bis 15 Glycerineinheiten und mindestens einer linearen oder verzweigten, gesättigten oder ungesättigten Fettsäure mit 6 bis 24 Kohlenstoffatomen; und
- Glyceryltrihydroxystearat;
wobei das Gewichtsverhältnis Polyglycerinester aus mindestens einer Poly(hydroxystearinsäure) und Carbonsäuren/Polyglycerinester mindestens einer Fettsäure strikt größer als 0,7, vorzugsweise größer als 1,0 ist.

2. Zusammensetzung nach Anspruch 1, **dadurch gekennzeichnet, dass** der Polyglycerinester aus mindestens einer Poly(hydroxystearinsäure) und Carbonsäuren aus der Veresterung zwischen einer Mischung aus Polyglycerin und (i) Poly(hydroxystearinsäure) mit 4 Polyglycerineinheiten; (ii) linearen oder verzweigten, aliphatischen Dicarbonsäuren mit 8 bis 12 Kohlenstoffatomen; und (iii) gesättigten oder ungesättigten, linearen oder verzweigten Fettsäuren mit 16 bis 20 Kohlenstoffatomen abgeleitet ist, und bevorzugt Polyglyceryl-4-diisostearat/Polyhydroxystearat/Sebacat ist.

3. Zusammensetzung nach Anspruch 1 oder 2, umfassend zwischen 0,5 und 5,0 Gew.-%, bevorzugt zwischen 1,0 und 4,0 Gew.-%, bevorzugter zwischen 1,5 und 3,5 Gew.-%, mindestens eines Polyglycerinesters von mindestens einer Poly(hydroxystearinsäure) und Carbonsäuren, bezogen auf das Gesamtgewicht der Zusammensetzung.

4. Zusammensetzung nach einem der vorhergehenden Ansprüche, **dadurch gekennzeichnet, dass** der Polyglycerinfettsäureester unter den Estern ausgewählt ist, die aus der Veresterungsreaktion zwischen einem Polyglycerin, das 4 bis 5 Glycerineinheiten und insbesondere 4 Glycerineinheiten umfasst, und mindestens einer aliphatischen Carbonsäure, die eine Alkylkette mit 16 bis 20 Kohlenstoffatomen, wie eine Stearyl- und/oder Isostearylkette, umfasst, abgeleitet sind.

5. Zusammensetzung nach einem der vorhergehenden Ansprüche, umfassend zwischen 0,1 und 5,0 Gew.-%, bevorzugt zwischen 0,5 und 4,0 Gew.-%, bevorzugter zwischen 1,0 und 4,0 Gew.-%, mindestens eines Polyglycerinfettsäureesters, bezogen auf das Gesamtgewicht der Zusammensetzung.

6. Zusammensetzung nach einem der vorhergehenden Ansprüche, umfassend zwischen 0,01 und 2,0 Gew.-%, insbesondere zwischen 0,1 und 1,5 Gew.-%, bevorzugt zwischen 0,1 und 1 Gew.-%, bevorzugter zwischen 0,2 und 0,8 Gew.-%, Glyceryltrihydroxystearat, bezogen auf das Gesamtgewicht der Zusammensetzung.

7. Zusammensetzung nach einem der vorhergehenden Ansprüche, umfassend zwischen 5,0 und 50 Gew.-% Fettphase, insbesondere zwischen 10 und 30 Gew.-% und vorzugsweise zwischen 15 und 25 Gew.-%, bezogen auf das Gesamtgewicht der Zusammensetzung.

8. Zusammensetzung nach einem der vorhergehenden Ansprüche, umfassend weniger als 2,0 Gew.-% Silikonöl(e), insbesondere weniger als 1,0 Gew.-% Silikonöl(e), bevorzugt weniger als 0,5 Gew.-% Silikonöl(e), bezogen auf das Gesamtgewicht der Zusammensetzung, und noch bevorzugter frei von Silikonöl(en) ist.

9. Zusammensetzung nach einem der vorhergehenden Ansprüche, die mindestens ein Öl auf Kohlenwasserstoffbasis enthält, das aus den flüchtigen linearen Alkanen mit 11 bis 13 Kohlenstoffatomen, insbesondere einem Gemisch aus Undekan und Tridekan, und den linearen und/oder verzweigten C₁₅ -C₁₉ Alkanen, insbesondere einem Gemisch aus linearen oder verzweigten C₁₅ -C₁₉ Alkanen, ausgewählt ist.

10. Zusammensetzung nach einem der vorhergehenden Ansprüche, umfassend mindestens ein nichtflüchtiges Öl auf Kohlenwasserstoffbasis, das aus synthetischen Ethern mit 10 bis 40 Kohlenstoffatomen, wie Dicaprylylether, Carbonaten, wie Dicaprylylcarbonat, Fettsäuretriglyceriden, insbesondere welchen, die gesättigt sind, wie Capryl-/Caprinsäuretriglycerid, und Gemischen davon ausgewählt ist.

11. Zusammensetzung nach einem der vorhergehenden Ansprüche, umfassend von 50 bis 90 Gew.-%, bevorzugt von 60 bis 85 Gew.-% und bevorzugter von 65 bis 75 Gew.-%, bezogen auf das Gesamtgewicht der Zusammensetzung, an wässriger Phase.

12. Zusammensetzung nach einem der vorhergehenden Ansprüche, ferner umfassend mindestens ein Konservierungsmittel, insbesondere eine Mischung von Konservierungsmitteln, die mindestens einen Alkohol, bevorzugt Ethanol, mindestens ein Glykol, bevorzugt Pentylenglykol, Salicylsäure, Kaliumsorbat und Phytinsäure umfasst.

13. Zusammensetzung nach einem der vorhergehenden Ansprüche, ferner umfassend mindestens ein von den Polyglycerinestern verschiedenes emulgierendes Tensid, das bevorzugt aus Glycerinestern von C₈ -C₂₄ Fettsäuren, insbesondere C₁₂-C₂₂ Fettsäuren, wie dem Glycerinester der Stearinsäure, ausgewählt ist.

14. Zusammensetzung nach einem der vorhergehenden Ansprüche, ferner umfassend mindestens einen Füllstoff enthält, der insbesondere aus Lauroyl-Lysin, Talk, Calciumcarbonat und Mischungen davon ausgewählt ist.

15. Zusammensetzung nach einem der vorhergehenden Ansprüche, ferner umfassend mindestens ein Polyol, das insbesondere aus Glycerin, Propandiol und Mischungen davon ausgewählt ist.

16. Zusammensetzung nach einem der vorhergehenden Ansprüche, **dadurch gekennzeichnet, dass** sie eine Viskosität im Bereich von 0,1 bis 10 Pa.s, bevorzugt von 0,5 bis 8,0 Pa.s, bevorzugter von 2,5 bis 7,0 Pa.s aufweist.

17. Kosmetisches Verfahren zur Pflege von Keratinmaterialien, insbesondere der Haut, umfassend mindestens einen Schritt des Auftragens einer Zusammensetzung nach einem der Ansprüche 1 bis 16 auf die Keratinmaterialien.

## Revendications

1. Composition, notamment une composition cosmétique, en particulier de maquillage et/ou de soin de matériaux kératiniques, comprenant :
- au moins une phase grasse continue ;
- au moins une phase aqueuse à une concentration d'au moins 50,0 % en poids, par rapport au poids total de la composition, et dispersée dans ladite phase grasse ;
- au moins un ester de polyglycérol d'au moins un poly(acide hydroxystéarique) et d'acides carboxyliques, issu de l'estérification entre au moins un polyglycérol et (i) au moins un poly(acide hydroxystéarique) ; (ii) au moins un acide di- et/ou tricarboxylique ; et (iii) au moins un acide gras saturé ou insaturé, linéaire ou ramifié ayant de 6 à 22 atomes de carbone ;
- au moins un ester de polyglycérol comprenant de 4 à 15 motifs de glycérol et au moins un acide gras, linéaire ou ramifié, saturé ou insaturé, comprenant de 6 à 24 atomes de carbone ; et
- du trihydroxystéarate de glycéryle ;
le rapport en poids d'ester de polyglycérol d'au moins un poly(acide hydroxy stéarique) et d'acides carboxyliques/ester de polyglycérol d'au moins un acide gras étant strictement supérieur à 0,7, de préférence supérieur à 1,0.

2. Composition selon la revendication 1, **caractérisée en ce que** l'ester de polyglycérol d'au moins un poly(acide hydroxystéarique) et d'acides carboxyliques est issu de l'estérification entre un mélange de polyglycérol et (i) de poly(acide hydroxystéarique) avec 4 motifs de polyglycérol ; (ii) d'acides dicarboxyliques aliphatiques, linéaires ou ramifiés, ayant 8 à 12 atomes de carbone ; et (iii) d'acides gras saturés ou insaturés, linéaires ou ramifiés ayant de 16 à 20 atomes de carbone, et est de préférence le diisostéarate / polyhydroxystéarate / sébacate de polyglycéryle-4.

3. Composition selon la revendication 1 ou 2, comprenant entre 0,5 % et 5,0 % en poids, de préférence entre 1,0 % et 4,0 % en poids, plus préférablement entre 1,5 % et 3,5 % en poids, d'au moins un ester de polyglycérol d'au moins un poly(acide hydroxystéarique) et d'acides carboxyliques, par rapport au poids total de la composition.

4. Composition selon l'une quelconque des revendications précédentes, **caractérisée en ce que** l'ester d'acide gras de polyglycérol est choisi parmi des esters issus de la réaction d'estérification entre un polyglycérol comprenant de 4 à 5 motifs de glycérol, en particulier comprenant 4 motifs de glycérol, et au moins un acide carboxylique aliphatique comprenant une chaîne alkyle ayant de 16 à 20 atomes de carbone, telle qu'une chaîne stéaryle et/ou isostéaryle.

5. Composition selon l'une quelconque des revendications précédentes, comprenant entre 0,1 % et 5,0 % en poids, de préférence entre 0,5 % et 4,0 % en poids, plus préférablement entre 1,0 % et 4,0 % en poids, d'au moins un ester d'acide gras de polyglycérol, par rapport au poids total de la composition.

6. Composition selon l'une quelconque des revendications précédentes, comprenant entre 0,01 % et 2,0 % en poids, notamment entre 0,1 % et 1,5 % en poids, de préférence entre 0,1 % et 1 % en poids, plus préférablement entre 0,2 % et 0,8 % en poids, de trihydroxystéarate de glycéryle, par rapport au poids total de la composition.

7. Composition selon l'une quelconque des revendications précédentes, comprenant entre 5,0 % et 50 % en poids de phase grasse, notamment entre 10 % et 30 % en poids, et de préférence entre 15 % et 25 % en poids, par rapport au poids total de la composition.

8. Composition selon l'une quelconque des revendications précédentes, comprenant moins de 2,0 % en poids d'huile(s) de silicone, notamment moins de 1,0 % en poids d'huile(s) de silicone, de préférence moins de 0,5 % en poids d'huile(s) de silicone, par rapport au poids total de la composition, et plus préférablement étant exempte d'huile(s) de silicone.

9. Composition selon l'une quelconque des revendications précédentes, comprenant au moins une huile à base d'hydrocarbure choisie parmi des alcanes linéaires volatils comprenant de 11 à 13 atomes de carbone, notamment un mélange undécane-tridécane, et des alcanes en C₁₅-C₁₉ linéaires et/ou ramifiés, notamment un mélange d'alcanes en C₁₅-C₁₉ linéaires et/ou ramifiés.

10. Composition selon l'une quelconque des revendications précédentes, comprenant au moins une huile hydrocarbonée non volatile choisie parmi des éthers synthétiques ayant de 10 à 40 atomes de carbone, tels que l'éther dicaprylylique, des carbonates, tels que le carbonate de dicaprylyle, des triglycérides d'acides gras, en particulier ceux qui sont saturés, tels qu'un triglycéride caprylique/caprique, et leurs mélanges.

11. Composition selon l'une quelconque des revendications précédentes, comprenant de 50 % à 90 % en poids de phase aqueuse, de préférence de 60 % à 85 % en poids, et plus préférablement de 65 % à 75 % en poids, par rapport au poids total de ladite composition.

12. Composition selon l'une quelconque des revendications précédentes, comprenant en outre au moins un conservateur, en particulier un mélange de conservateurs comprenant au moins un alcool, de préférence l'éthanol, au moins un glycol, de préférence le pentylène glycol, l'acide salicylique, le sorbate de potassium et l'acide phytique.

13. Composition selon l'une quelconque des revendications précédentes, comprenant en outre au moins un tensioactif émulsifiant différent des esters de polyglycérol, de préférence choisi parmi des esters de glycérol d'acides gras en C₈-C₂₄, en particulier d'acides gras en C₁₂-C₂₂, tels que l'ester de glycérol d'acide stéarique.

14. Composition selon l'une quelconque des revendications précédentes, comprenant en outre au moins une charge, notamment choisie parmi la lauroyl-lysine, le talc, le carbonate de calcium et leurs mélanges.

15. Composition selon l'une quelconque des revendications précédentes, comprenant en outre au moins un polyol, notamment choisi parmi la glycérine, le propanediol et leurs mélanges.

16. Composition selon l'une quelconque des revendications précédentes, **caractérisée en ce qu'**elle présente une viscosité allant de 0,1 à 10 Pa.s, de préférence de 0,5 à 8,0 Pa.s, plus préférablement de 2,5 à 7,0 Pa.s.

17. Procédé cosmétique de soin de matériaux kératiniques, notamment la peau, comprenant au moins une étape d'application sur lesdits matériaux kératiniques d'une composition telle que définie dans l'une quelconque des revendications 1 à 16.
